# EUROPEAN PATENT APPLICATION

(11) **EP 1 090 647 A1**
(43) Date of publication of application: **11.04.2001**
(21) Application number: 99203297.9
(22) Date of filing: 07.10.1999
(51) Int. Cl.: A61L 9/04, A01N 25/04

(54) **Process for encapsulating active agents obtaining a gel**

(71) Applicant: Instituut voor Agrotechnologisch Onderzoek (ATO-DLO), 6700 AA Wageningen (NL)
(72) Inventor: Yilmoz, Gülden, 6671 AE Zetten (NL); Jongboom, Remigius Oene Jules, 6671 AE Zetten (NL); Oosterhaven, Jacobus, 6708 SR Wageningen (NL)
(74) Representative: de Bruijn, Leendert C.

(57) **Abstract**

The present invention relates to a process for encapsulating an active agent in a biopolymer in the form of a gel, comprising the steps of:
a) forming a dispersion or solution of the biopolymer in water; and
b) adding the active agent to the dispersion or solution obtained in step a);
wherein the biopolymer is at least partially dissolved before and/or after addition of the active agent.

The gels obtained with the present invention are particularly suitable for coating or impregnating packaging materials

## Description

The present invention relates to a process for encapsulating of an active agent in gels and to gels obtained therewith. The present invention also relates to the use of such gels as a release system on or in packaging materials (e.g. as a coating).

Controlled release of an active agent is required for many applications. Examples of such applications arc air refreshers, which release a fragrance over a certain period of time. Another example of an application is the release of volatile agrochemicals into the environment of use.

Formulations for controlled release of active agents, for instance volatile substances are known. The particular substances can be absorbed onto a carrier such as a synthetic resin for instance. Another possibility is encapsulation of the substance into microparticles and matrices. However, the processes for obtaining such products are often complicated and the release profiles obtained therewith are not always satisfactory. Further the amount of active agent that can be incorporated is most of the times rather low and losses during processing are very high.

The present invention relates to a process for encapsulating an active agent in a biopolymer in the form of a gel, comprising the steps of:
a) forming a dispersion or solution of the biopolymer in water; and
b) adding the active agent to the dispersion or solution obtained in step a);
wherein the biopolymer is at least partially dissolved before and/or after addition of the active agent.

The main advantages of this process are:
∗ The process can easily be adjusted by varying any of the process parameters to obtain certain structural and physical properties of the matrix biopolymer to adjust product properties.
∗ In these delivery systems loads can reach values up to 60-70 % (w/w, of the composition) of active agent.
∗ The delivery systems can efficiently be prepared via an extrusion process.

As the biopolymer, any hydrophilic, biologically degradable polymer that is swellable or dissolvable in water or in water containing mixtures can be used. Examples thereof are starch, starch derivatives, gum, gelatine, cellulose derivatives, alginates, pectins, carragenans etc. Preferably, native potato starch or pre-gelatinised starch is used.

The biopolymer concentration is 2 to 90 wt.%, preferably 20 to 60 wt.%, based on the weight of the total composition.

As described above the biopolymer is partially dissolved before and/or after addition of the active agent. This means that the present invention encompasses three embodiments. According to a first embodiment the biopolymer is dissolved after addition of the active agent. According to a second embodiment the biopolymer is dissolved before addition of the active agent. According to a third embodiment the biopolymer is partially dissolved before addition of the active agent and further dissolved after addition of the active agent. It is preferred that the biopolymer is dissolved before addition of the active agent.

Dissolution of the biopolymer can be obtained by heating the biopolymer to an elevated temperature. In particular, heating the biopolymer to a temperature around 65°C is required when native potato starch is used as a biopolymer. In case of native potato starch, dissolution of the polymer is often referred to as gelatinization. Other possibilities can be addition of sodium hydroxide to native potato starch for gelatinization.

According to the present invention the solution of dispersion of the biopolymer is also meant to include other physical states of the biopolymer, such as a water containing melt of the polymer. A water containing melt of the biopolymer is used when extrusion is applied, as will be explained later.

As the active agent, compounds ranging from water soluble to water insoluble, organic to inorganic, volatile -non volatile or mixtures of compounds which can be in solid, liquid or gas form, such as fragrances, pheromones, essential oils, herbicides, rodenticides, etc can be used. The present process is particularly useful when the active agent is a volatile substance.

Examples of these substances are agrochemicals, for instance herbicides, and fragrances.

The active agent is added to the dispersion or solution of the biopolymer in an amount of 5 to 70 wt.%, preferably 10 to 60 wt.%, based on the total composition.

The active agent can be added to the dispersion or solution of the biopolymer as a pure compound, in the form of an emulsion or as a dispersion of the active agent. When an emulsion is used, preferably, the ratio between active agent and water in the emulsion is 10:90 to 50:50 (w/w). The active agent itself can be added directly to the water or it can be mixed in a suitable hydrophobic substance. Examples of suitable hydrophobic substances are all water-immiscible liquids, solids such as hydrocarbons (alkanes, cycloalkenes), ethers, esters, halogenated hydrocarbons and oils.

An emulsifier can be added to the emulsion of a hydrophobic active agent in water. The emulsifier can be a cationic, anionic, and non-ionic surface active agent of animal, plant or mineral origin. Examples of emulsifiers are polyoxyethylene sorbitan esters, sucrose esters of fatty acids, propylene glycol esters, polyglycerol esters of fatty acids and lecithins. Preferably Tween 20, 40, 60, 65 and 80 are used. The emulsifier can be added to the emulsion in an amount of 0.1 to 50 % (w/w), preferably 0.5 to 25 % (w/w) based on the active agent.

From the mixture of active agent and water, a stable emulsion can be formed by applying adequate mechanical forces. Equipment, which can be used for this, is for example an Ultra-Turrax, a top stirrer, a homogeniser or any other suitable emulsion-forming equipment.

When the active agent is added in the form of an emulsion or dispersion to the biopolymer dispersion or solution, the ratio of biopolymer phase to active agent phase is 10/90 to 70/30 (w/w).

To the solution or dispersion of the biopolymer also a plasticizing agent (other than water) can be added. Example of plasticizing agents are glycerol, polyhydric alcohols, ethylene glycol, glucose, sorbitol, propylene glycols, maltose alditols, glyol glycosides, polyglycerols, amino alcohols, and amides. The plasticizing agent serves to adjust the processability, permeability of the matrix polymer, and mechanical properties The plasticizing agent is added in an extra amount of 20 to 40 wt.%, more preferably 25-35 wt.%, based on dry biopolymer.

Addition of a fungicide or a biocide to the formulation can be considered in order to prevent that the biological degradation of the biopolymer proceeds too fast.

The active agent and the dispersion and solution of the biopolymer can be mixed using conventional mixing devices, such as a static mixer, homogenizer, ultra-turrax, blade mixer, kneader etc. or by using continuous processing equipment like extruders.

When an extruder is used for processing the biopolymer the biopolymer, plasticizer, water emulsifier and, the active agent can be fed independently. If the active agent is fed in the form of an emulsion, there is no need to add extra emulsifier to the extruder.

Water is fed in the feeding zone or in the first zone (after the feeding zone) in an amount of 20 to 40 %, preferably 30 to 35 %, based on dry biopolymer. Active agent is injected downstream in the extruder. It is preferred that active agent is injected when the biopolymer is dissolved or molten. During extrusion the melt temperature should preferably not exceed 120 °C at the die when volatiles are used in order to prevent foam formation.

As described above the gels according to the present invention are very advantageous in that they can contain up to 70 % by weight of the active agent. The gel according to the invention can be processed further into the form of a film, powder, granulate, hydrogel, shaped article, continuous article impregnated on a carrier or as a coating on a packaging material.

Usage of co-extrusion can be performed for enabling the device to be processed together with the packaging material on which the device would be applied as a coating or with the carrier on which the gel will be impregnated.

Cross linking of the matrix biopolymer can be performed when necessary. As cross-linking agents conventionally, bi- or multifunctional reactants are used. When Starch is used as the biopolymer, examples for crosslinking agents are epichlorohydrine (EPC), glyoxal, trisodium trimetaphosphate (TSTP), phosphorylchloride or di- or polybasic carboxylic acid anhydrides and etc. Preferably, epichlorohydrine or trisodium trimetaphosphate is used. Cross-linking can be induced by means of a suitable catalyst, such as a base, acid or salt. In case of trisodium trimetaphosphate, sodium hydroxide can be used as a catalyst.

Application of a hydrophobic coating on the device can be considered. The hydrophobic coating can be of animal, plant or synthetic origin. It can be solid or liquid. The hydrophobic coating layer can be considered to prevent dehydration of the gel in low humid environment or provide a barrier to the environmental conditions, which provide degradation.

The delivery device can be designed to give zero order, first order release patterns or to give triggered, burst delayed or sustained release. Release pattern and rate of the active ingredient encapsulated or the degradation characteristics of the matrix biopolymer can be adjusted by changing the structural characteristics of the matrix biopolymer or the composition of the device.

In matrix systems as in the invention the active agent is homogeneously dissolved or dispersed throughout the polymer mass. The release pattern depends upon the permeability of the matrix polymer, active agent (load and solubility) and the geometry of the device.

The principle steps involved in the release of the active agent depend on whether the active agent is dissolved or dispersed (related to water solubility and water miscibility). The steps involved in the release of an active agent from the biopolymer matrix that is dispersed are dissolution into the matrix, diffusion within the matrix and diffusion into the environment.

In the first step, the active agent has to pass through the barrier of emulsifier around the dispersed phase of the active agent. Changing the type of emulsifier or the amount of emulsifier can alter the effect of this barrier. Additionally the dispersed phase morphology can be considered as it affects the interfacial area between the matrix and the active agent. In the second step the diffusion of the active agent within the matrix polymer takes place which relates to the permeability of the matrix biopolymer.

Type of the matrix biopolymer or the concentration of the biopolymer can be altered to give the required permeability or induce crystallinity (provides additional barriers for diffusion). Addition of a plasticizer is also a tool that is affecting the permeability of the matrix as it relates to the physical state of the matrix and thus the mobility of the polymer chains. Cross-linking of the matrix polymer, also helps adjusting the permeability by changing density of the biopolymer network.

In the third step diffusion into the environment takes place. Here the matrix geometry and application of an additional coating gains importance. Application of a coating resembles an additional barrier in the last step and it can also help to prevent dehydration of the system keeping the mobility of the matrix.

When the active agent is dissolved the steps involved in the release are diffusion of the active agent to the surface of the matrix and diffusion into the environment. In this case the parameters which affect the matrix permeability gain importance as mentioned before (type or concentration of the biopolymer, addition of plasticizers, cross-linking and etc.). The matrix geometry and application of a coating is again important parameters to affect the release of the active agent.

The release of an active agent from a matrix can be by diffusion as explained before or by biodegradation or the combination of these two. The biodegradation of the matrix biopolymer depends on the structural characteristics of the polymer. The biodegradation of the matrix can be altered by changing the type, concentration of the biopolymer, addition of plasticizers and crosslinking and the application of a coating which will provide a barrier to environmental conditions which provides degradation.

The modification of physical properties like rigidity of the matrix can also be achieved by cross-linking of the matrix polymer, addition of plasticizer, water content of the system, crystallinity and the molecular weight of the polymer.

The gels obtained according to the present invention can be applied as a coating onto packaging materials. This application is especially suitable if the active agent is a bio active compound. The packaging materials can then be used for packaging fruits, vegetables and flowers, for instance. The active agent can for instance be carvone, as a sprout inhibiting agent.

Hereafter some examples of the invention are given which should not be construed as a limitation of the scope of the invention. The examples are further illustrated in the drawings, wherein
figure 1 shows the release profile of an active substance according to examples 1 to 3 and
figure 2 shows a release profile of an active substance according to exampl es 5 to 8.

### Example 1

20 grams of Flocgel (destructurized potato starch, moisture content 10% w/w, obtainable from Avebe, NL) was mixed with 200 ml of demi water at room temperature obtaining a gel. 5 grams of TSTP (trisodium trimetaphosphate) was added to the gel.
15 grams of S-carvone as the volatile substance, was mixed with 5 grams of paraffin oil. This mixture was emulsified in water containing 1 g Tween 80, obtaining a fine dispersion. This dispersion was mixed with the starch gel obtaining a homogeneous mixture. To this mixture 10 ml of NaOH solution was added in order to start the cross-linking reaction of the starch. This dispersion was casted onto a glass tray and closed to avoid drying of the gel.

### Example 2

Example 1 was repeated, with the exception that S-carvone was not mixed with paraffin oil.

### Example 3

Example 1 was repeated, with the exception that 40 grams of S- carvone were used.

### Example 4

10 grams of Flocgel were mixed with 200 ml of demi water at room temperature obtaining a gel. 20 grams of native potato starch were added to the gel.
50 grams of S-carvone as the volatile substance were emulsified in water containing 1 g Tween 80, obtaining a fine dispersion. This dispersion was mixed with the starch gel obtaining a homogeneous mixture. This mixture was put in glass cups. A heat treatment was performed (1 to 4.5 hours) in order to gelatinise suspended native starch in the dispersion. During this process the sample was closed to prevent evaporation of S-carvone.

### Example 5

A 2% starch solution was prepared by mixing 2 grams of native potato starch with 100 ml of demi water and the solution was dissolved by applying heat. This solution was cooled down to room temperature and 2.8 grams or native potato starch was suspended in the solution using a propeller mixer.
An emulsion of active agent (s-carvone) was prepared by mixing 10 grams of demi water, 8 grams of s-carvone and 4 grams of Tween 20, using an ultra turrax. This emulsion was added to the starch solution and the mixture obtained was put in glass cups. Heat was applied to the closed cups (70 °C for 1,5 hr). A plant origin fat coating (biskien fat) was applied on the surface.

### Example 6

Example 5 was repeated with the exception that 4.8 g native starch was added to the 2 % starch solution.

### Example 7

Example 4 was repeated with the exception that 3.6 grams additional water was added and the active agent was mixed tot he 2% starch solution after suspending the native starch by using a propeller mixer.

### Example 8

Example 7 was repeated with the exception that 4.8 g native starch was added to the 2 % starch solution.

### Example 9

This example relates to preparation of a gel by means of an extruder. A premix consisting of native potato starch (18 % moisture), glycerol (20 % based on dry starch, w/w), Tween 20 (5 % based on the active agent to be encapsulated) was prepared. Water was fed together with the feed of the premix in an amount of 30 % based on dry starch (w/w). Active agent (carvone) (35 %, based on dry starch, w/w) was injected in the 7th zone.

### Example 10

This example relates to preparation of a gel by means of an extruder. A premix consisting of native potato starch (18 % moisture), glycerol (20 % based on dry starch, w/w) was prepared. Water was fed together with the feed of the premix in an amount of 20 % based on dry starch (w/w). An emulsion of active agent (carvone) in water, consisting of 45 % active agent and 5 % Tween 20 (10 % based on the weight of the active agent, w/w), prepared by means of an ultra turrax was injected in the 7th zone (40 % based on dry starch, w/w).

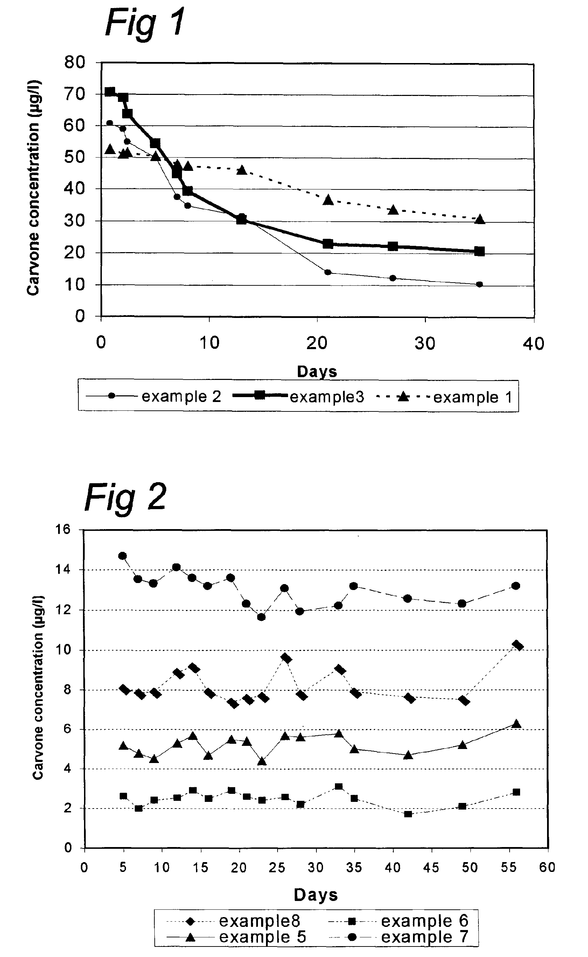

Release experiments for some samples (examples 1 to 3 (figure 1) and examples 5 to 8 (figure 2)) were performed using a static headspace analysing method. Analysis were performed in exsiccators, where the samples were placed in The carvone content in the air was determined taking 10 ml. air samples 3 times a week. After sampling the exsiccators were open for one hour. The analysis was carried out at a storage temperature of 12°C. The release of carvone for the examples can be seen in figure 1 and figure 2.

## Claims

**1.** Process for encapsulating an active agent in a biopolymer in the form of a gel, comprising the steps of:
a) forming a dispersion or solution of the biopolymer in water; and
b) adding the active agent to the dispersion or solution obtained in step a);
wherein the biopolymer is at least partially dissolved before and/or after addition of the active agent.

**2.** Process according to claim 1, wherein the biopolymer concentration is 2 to 90 wt.%, preferably 20 to 60 wt.%, based on the weight of the total composition.

**3.** Process according to claim 1 or 2, wherein the active agent is added to the dispersion or solution obtained in step a) as a pure compound, in the form of an emulsion, or dispersion of the active agent in water.

**4.** Process according to claim 3, wherein the acitive agent is added in the form of an emulsion and the ratio active agent to water in the emulsion is 10:90 to 50:50.

**5.** Process according to any of the preceding claims, wherein the biopolymer is starch or a derivative thereof, preferably native potato starch or pre-dissolved potato starch.

**7.** Process according to any of the proceeding claims, wherein the active agent is mixed with the aqueous dispersion or solution of the biopolymer in an extruder.

**8.** Process according to any of the preceding claims, wherein the active agent is a volatile substance.

**9.** Process according to claim 9, wherein the active agent is a fragrance or an agrochemical.

**10.** Process according to any of the proceeding claims, which further comprises the step of applying a coating onto the gel.

**11.** Gel comprising a biopolymer and an active agent obtainable according to the process according to any of the preceding claims.

**12.** Gel according to claim 11, containing 5 to 70 wt%, based on total composition, of the active agent.

**13.** Use of a gel according to claim 1 of 12 for coating or impregnating packaging materials.

**14.** Use of a gel according to claim 13, wherein the active agent is a bioactive compound, for coating or impregnating packaging materials for vegetables, fruit or flowers.
